(19) Europäisches Patentamt
European Patent Office
Office européen des brevets

(11) Numéro de publication: **0 097 564 B1**

(12) # FASCICULE DE BREVET EUROPEEN

(45) Date de publication du fascicule du brevet: **13.11.85**

(51) Int. Cl.⁴: **C 07 C 69/157**, C 07 C 67/29, C 07 C 68/06, C 07 C 103/00

(21) Numéro de dépôt: **83401154.6**

(22) Date de dépôt: **07.06.83**

(54) **Procédé d'hydroxylation par le peroxyde d'hydrogène en milieu superacide de dérivés du phénol et de l'aniline.**

(30) Priorité: **18.06.82 FR 8210644**

(43) Date de publication de la demande: **04.01.84 Bulletin 84/1**

(45) Mention de la délivrance du brevet: **13.11.85 Bulletin 85/46**

(84) Etats contractants désignés: **BE DE FR GB IT NL**

(56) Documents cités: **neant**

(73) Titulaire: **ATOCHEM, 12/16, allée des Vosges, F-92400 Courbevoie (FR)**

(72) Inventeur: **Morellet, Guy, 32 rue des Carmes, F-86000 Poitiers (FR)**
Inventeur: **Jacquesy, Jean-Claude, 46, rue du Planty Buxerolles, F-8600 Poitiers (FR)**
Inventeur: **Jouannetaud, Marie-Paule, 8, Allée du Nivernais, F-86000 Poitiers (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 097 564 B1

**Description**

La présente invention concerne un procédé d'hydroxylation par le peroxyde d'hydrogène en milieu superacide de dérivés du phénol et de l'aniline, répondant à la formule générale:

$$C_6H_5-X-\underset{\underset{O}{\parallel}}{C}-R$$

Dans cette formule X représente soit un atome d'oxygène, soit un groupement — NH — et R représente un atome d'hydrogène, ou un groupement alkyle contenant de 1 à 8 atomes de carbone, ou un groupement cycloalkyle contenant de 5 à 10 atomes de carbon, ou un groupement aryle, éventuellement substitué, ou un groupement alcoxy contenant de 1 à 8 atomes de carbone, ou un groupement cycloalcoxy contenant de 5 à 10 atomes de carbone, ou encore un groupement aryloxy.

Le procédé objet de l'invention permet d'introduire sur le noyau phényle une fonction phénol en positions ortho, méta et para par rapport à la fonction déjà présente sur le cycle. La proportion de l'isomère méta-hydroxylé est toujours importante, voire prépondérante.

Les composés aromatiques susceptibles d'être hydroxylés par le procédé selon l'invention sont donc:

- les esters du phénol avec les mono- et polyacides carboxyliques aliphatiques, cycloaliphatiques et aromatiques,
- le carbonate de bis-phényle et les carbonates mixtes de phényle et d'alkyle ou de cycloalkyle ou d'aryle substitué,
- les anilides,
- les phénylcarbamates d'alkyle, de cycloalkyle et d'aryle.

Dans les procédés connus d'hydroxylation radicalaire du phénol et des éthers phénoliques par le réactif de FENTON (peroxyde d'hydrogène et ions ferreux) ou ses analogues, l'hydroxylation se fait préférentiellement en ortho et en para de la fonction phénol. Le taux d'hydroxylation en position méta est toujours faible. De tels procédés sont décrits par exemple dans l'ouvrage de HOUBEN-WEYL, »Methoden der organischen Chemie«, Vierte Auflage, Vol. 6/1 C, pages 30 à 34.

De même l'hydroxylation radicalaire de l'acétanilide conduit à un mélange constitué essentiellement de dérivés ortho- et para-hydroxylés, le dérivé méta-hydroxylé étant très minoritaire. Ces travaux sont exposés par exemple dans les articles de D. I. METELITSA, Russian Chemical Reviews, 40 (7), 1971, pages à 580, et de J. VARAGNAT, Ind. Eng. Chem., Prod. Res. Dev., 15 (3), 1976, pages 212 à 215.

L'hydroxylation ionique des phénols au moyen de peroxyde d'hydrogène et d'acides carboxyliques, en présence d'acide minéral fort, a également été étudiée. L'acide percarboxylique formé intermédiairement est un oxydant très puissant, qui conduit surtout aux dérivés dihydroxylés en ortho et en para. Il ne se forme pratiquement pas d'isomère méta.

D'autres procédés d'hydroxylation ionique du phénol, catalysée par des acides minéraux forts, font intervenir des ions peroxonium. Ils conduisent à un mélange de pyrocatéchol et d'hydroquinone, sans formation de résorcinol. Ces procédé sont décrits dans les références déjà citées et dans le numéro de »Chimie Actualités« du 3 novembre 1976, pages 47 à 49.

L'hydroxylation de certains hydrocarbures aromatiques par le peroxyde d'hydrogène en milieu superacide, rapportés par G. A. OLAH et R. OHNISHI dans J. Org. Chem., 43 (5), 1978, pages 865 à 867, conduit à des dérivés mono-hydroxylés. Ainsi le benzène fournit le phénol avec un rendement de 67%, tandis que le toluène fournit, avec un rendement de 67% aussi, un mélange de crésols dans le rapport ortho/méta/para = 71/6/23.

Selon la demande de brevet français N° 2 483 403, au nom de la demanderesse, l'hydroxylation d'alkylphénols et de leurs éthers par le peroxyde d'hydrogène en milieu superacide conduit préférentiellement au dérivé métahydroxylé. Mais, dans les mêmes conditions, le phénol ordinaire ne réagit que très partiellement pour conduire à un mélange constitué principalement d'hydroquinone et de pyrocatéchol; il ne se forme que très peu de résorcinol.

C'est donc de manière tout-à-fait inattendue au vu de l'art antérieur que la demanderesse a découvert que les esters du phénol avec les acides carboxyliques, le carbonate de bis-phényle et les carbonates mixtes de phényle et d'alkyle ou de cycloalkyle ou d'aryle substitué, les anilides et les phénylcarbamates d'alkyle, de cycloalkyle et d'aryle peuvent être hydroxylés sur le noyau phényle par le peroxyde d'hydrogène en milieu superacide liquide pour conduire à un mélange de dérivés hydroxylés en positions ortho, méta et para par rapport à la fonction déjà existante sur le cycle. Dans ce mélange l'isomère méta est toujours présent en proportion importante, parfois même majoritaire.

Il a été observé que, lorsqu'on met en œuvre le carbonate de bis-phényle, dérivé symétrique à deux noyaux aromatiques non-substitués, la réaction d'hydroxylation ne porte que sur l'un des noyaux phényle.

Dans le cas du benzoate de phényle, le noyau porteur de la fonction acide n'est pas touché par

l'hydroxylation.

Dans le cas du benzanilide et du phénylcarbamate de phényle, c'est le cycle azoté qui réagit seul.

La réaction d'hydroxylation des esters phénoliques selon l'invention peut parfois s'accompagner d'une coupure partielle ou totale de la fonction ester, conduisant ainsi directement à la formation de diphénols. Mais de toutes façons les produits de la réaction peuvent être facilement transformés, par des moyens connus, en pyrocatéchol, résorcinol et hydroquinone, qui sont des matières premières importantes pour la synthèse organique.

Le milieu superacide liquide dans lequel se déroule la réaction est constitué de produits qui, sur l'échelle logarithmique de HAMMETT, ont des acidités atteignant environ — 25. A titre de comparaison, l'acide sulfurique à 100% a une acidité de — 11 et l'acide fluorhydrique anhydre une acidité de — 10. Les superacides liquides utilisés ont donc des acidités jusqu'à $10^{14}$ fois plus élevées que celles des acides minéraux forts habituels. Comme exemples de superacides liquides utilisables dans le procédé selon l'invention, on peut citer »l'acide magique« $FSO_3H-SbF_5$, l'acide fluoroantimonique $HF-SbF_5$, le complexe acide trifluorométhanesulfonique-pentafluorure d'antimoine $CF_3SO_3H-SbF_5$, et le complexe acide fluorhydriquetrifluorure de bore $HF-BF_3$.

Le peroxyde d'hydrogène est mis en œuvre sous forme de solutions aqueuses commerciales, dont le titre peut aller de 20 à 98% en poids.

La proportion de superacide par rapport au substrat à hydroxyler peut varier dans de larges limites. Elle est en général d'autant plus élevée que la solution de peroxyde d'hydrogène est plus diluée. On utilise le plus souvent un excès de peroxyde d'hydrogène par rapport à la stoechiométrie.

La réaction se déroule à la pression atmosphérique et à des températures allant de —80° C environ à 0° C environ. Elle est rapide et dure le plus souvent de quelques minutes à une heure.

Le mode opératoire général suivi pour mettre en œuvre le procédé selon l'invention est très simple: dans un récipient en poly(tétrafluoréthylène), ou en acier inoxydable lorsqu'on opère avec les complexes $HF-BF_3$, muni d'une agitation magnétique et refroidi à la température désirée, on introduit successivement le superacide liquide, le substrat à hydroxyler et la solution aqueuse de peroxyde d'hydrogène. Le mélange est homogénéisé et maintenu pendant le temps désiré à la température choisie. Il est ensuite versé sur un mélange d'eau, de glace et de carbonate acide de sodium. Après neutralisation, la solution est extraite plusieurs fois à l'éther diéthylique. La phase organique est lavée à l'eau jusqu'à neutralité, puis séchée sur du sulfate de sodium anhydre. Après évaporation de l'éther, le produit brut de la réaction est filtré sur un gel de silice et analysé par chromatographie en phase vapeur ou par chromatographie en phase liquide à haute pression.

Selon une variante de ce mode opératoire général, on peut introduire le peroxyde d'hydrogène dans le superacide liquide avant le substrat à hydroxyler. Selon d'autres variantes, convenant notamment pour les mélanges $HF-BF_3$, on introduit dans le réacteur d'abord le substrat, puis le superacide et le peroxyde d'hydrogène, ou le substrat, puis le peroxyde d'hydrogène et le superacide.

Les exemples suivants, donnés à titre non-limitatif, illustrent divers aspects de la mise en œuvre du procédé selon l'invention.

## Exemple 1

### Hydroxylation de l'acétate de phényle en milieu $HF-SbF_5$

a) Suivant le mode opératoire général, on refroidit à —40° C 20 ml d'un mélange $HF-SbF_5$ 1,75 M, avec un rapport molaire $SbF_5/HF = 0{,}04$. On ajoute 4 millimoles d'acétate de phényle et 2 équivalents de peroxyde d'hydrogène sous forme d'une solution aqueuse à 70% en poids. La réaction dure 30 minutes. Après traitement, les produits sont chromatographiés sur gel de silice en utilisant comme solvant d'élution un mélange de 15 parties en volume d'acétate d'éthyle et de 85 parties en volume d'éther de pétrole.

b) On opère comme ci-dessus, mais en utilisant une solution aqueuse à 95% en poids de peroxyde d'hydrogène.

Dans les deux cas, l'analyse spectroscopique (infra-rouge, résonance magnétique nucléaire, spectrographie de masse) et l'identification à des produits préparés montrent que les produits de la réaction sont constitués, à côté d'un peu d'acétate de phényle non transformé, des trois isomères ortho, méta et para de l'acétate d'hydroxyphényle, c'est-à-dire par les monoacétates du pyrocatéchol, du résorcinol et de l'hydroquinone.

Les résultats quantitatifs de ces deux essais sont consignés dans le Tableau 1.

Tableau 1

| Essai | Taux de transformation en produits hydroxylés | Proportions relatives des isomères ortho | méta | para |
|---|---|---|---|---|
| 1a | 86% | 6 | 51 | 43 |
| 1b | 87% | 6 | 64 | 30 |

Exemple 2

Hydroxylation de l'acétate de phényle en milieu $HF-BF_3$

a) On introduit dans le réacteur 25 millimoles d'acétate de phényle que l'on refroidit à −60° C. On fait le vide et on introduit successivement 1,5 mole d'acide fluorhydrique anhydre et 1,5 mole de trifluorure de bore. A l'aide d'une pompe on additionne ensuite 2 équivalents de peroxyde d'hydrogène sous forme d'une solution aqueuse à 70% en poids. Après trente minutes de réaction à −50° C sous agitation, les produits de la réaction sont traités comme indiqué dans le mode opératoire général et analysés par chromatographie en phase gazeuse et par chromatographie en phase liquide.

b) On opère comme ci-dessus, mais en introduisant le peroxyde d'hydrogène avant l'acide fluorhydrique et le trifluorure de bore.

Dans les deux cas, l'analyse spectroscopique et la comparaison à des produits connus montrent que le produit brut de la réaction, contient de l'acétate de phényle non transformé, du phénol ordinaire et les trois isomères ortho, méta et para de l'acétate d'hydroxyphényle.

Le tableau 2 rassemble les résultats quantitatifs de ces essais (% en poids).

Tableau 2

| Essai | Acétate de phényle non transformé | Phénol | Acétates d'hydroxy-phényle | Proportions relatives des isomères ortho | méta | para |
|---|---|---|---|---|---|---|
| 2a | 64 | 22 | 14 | 6 | 50 | 44 |
| 2b | 63 | 9 | 28 | 14 | 39 | 47 |

Exemple 3

Hydroxylation de l'acétate de phényle en milieu $FSO_3H-SbF_5$

a) Dans un flacon en poly(tétrafluoréthylène) refroidi à −40° C on verse 6 ml de $FSO_3H$, puis 3 ml de $SbF_5$ (rapport molaire $SbF_5/FSO_3H = 0{,}4$). On ajoute 2 millimoles d'acétate de phényle, puis 2 équivalents de peroxyde d'hydrogène, sous forme d'une solution aqueuse à 70% en poids. Le mélange est agité pendant 25 minutes à −40° C, puis hydrolysé et extrait. Le produit brut obtenu (100 mg) est analysé par chromatographie en phase gazeuse.

b) On opère comme ci-dessus, mais on introduit le peroxyde d'hydrogène avant l'acétate de phényle. La durée de réaction est de 20 minutes. On obtient après traitement 120 mg de produit brut.

Le tableau 3 donne les rendements en poids des produits analysés.

Tableau 3

| Essai | Acétate de phényle non transformé | Phénol | Acétates d'hydroxyphényle (isomères 3 et 4) | Résorcinol | Hydroquinone |
|---|---|---|---|---|---|
| 3a | 2 | 5,5 | 2 | 19,5 | 16,5 |
| 3b | — | — | 6,5 | 22 | 23 |

Exemple 4

Hydroxylation de l'acétate de phényle en milieu $CF_3SO_3H-SbF_5$

On verse dans le réacteur refroidi à —40°C 6 ml d'acide trifluorométhanesulfonique et 3 ml de pentafluorure d'antimoine (rapport molaire $SbF_5/CF_3-SO_3H$ = 0,6). Après homogénéisation de la solution, on ajoute successivement 1,8 équivalent de peroxyde d'hydrogène sous forme d'une solution aqueuse à 70% en poids, et 2 millimoles d'acétate de phényle. Le mélange est agité pendant 25 minutes à —40°C, puis hydrolysé et extrait. On récupère 125 mg de produits bruts, qui sont analysés par chromatographie en phase gazeuse et chromatographie en phase liquide sous haute pression.

Les résultats des analyses (rendements pondéraux) sont indiqués dans le tableau 4.

Tableau 4

| Acétate de phényle non transformé | Phénol | Diacétate de l'hydroquinone | Acétates d'hydroxyphényle (isomères 3 et 4) | Résorcinol | Hydroquinone |
|---|---|---|---|---|---|
| 1 | 2 | 3,5 | 20 | 10 | 11 |

Les acétates d'hydroxyphényle sont un mélange contenant 7% d'isomère méta et 93% d'isomère para.

Exemple 5

Hydroxylation du formiate de phényle en milieu $HF-SbF_5$

A 16 ml d'un mélange $HF-SbF_5$ 1,75 M (rapport molaire $SbF_5/HF$ = 0,04), refroidi à —20°C, on ajoute 4,18 millimoles de formiate de phényle, puis 1,5 équivalent de peroxyde d'hydrogène sous forme d'une solution aqueuse à 70% en poids. Le mélange réactionnel est maintenu pendant 30 minutes à —20°C puis traité comme indiqué dans le mode opératoire général.

Le produit brut réaction est filtré sur 60 g de gel de silice. Le phénol ordinaire formé est élué par un mélange 85/15 en volumes d'éther de pétrole et d'éther diéthylique. Un mélange 70/30 en volumes d'éther de pétrole et d'éther diéthylique élue le pyrocatéchol. Un mélange 65/35 des mêmes solvants élue un mélange de formiates d'hydroxyphényle. Un mélange 55/45 des mêmes solvants élue successivement le résorcinol et l'hydroquinone. Le tableau 5 donne les rendements pondéraux des produits de la réaction.

Tableau 5

| Phénol | Pyrocatéchol | Formiates d'hydroxyphényle | Résorcinol | Hydroquinone |
|---|---|---|---|---|
| 4,5% | 10,5% | 14,5% | 28,5 | 26% |

Exemple 6

Hydroxylation du carbonate de méthyle et de phényle en milieu $HF-SbF_5$

a) A 12 ml d'un mélange $HF-SbF_5$ 1,5 M (rapport molaire $SbF_3/HF$ = 0,0375), refroidi à —78°C on ajoute successivement 3 millimoles de carbonate de méthyle et de phényle et 1,5 équivalent de peroxyde d'hydrogène sous forme d'une solution aqueuse à 95% en poids. Le mélange est maintenu sous agitation et en l'espace d'une heure on laisse remonter la température à —10°C. Après traitement, les produits de la réaction sont chromatographiés sur gel de silice, en utilisant comme éluant un mélange 85/15 en volumes d'éther de pétrole et d'acétate d'éthyle.

b) On opère comme ci-dessus, mais en utilisant un mélange $HF-SbF_5$ 1,75 M (rapport molaire $SbF_5/HF$ = 0,04).

Dans les deux cas, les analyses spectroscopiques et la comparaison des produits séparés à des

produits connus montrent qu'il est formé les trois isomères ortho, méta et para du carbonate de méthyle et d'hydroxyphényle.

Le tableau 6 donne les résultats quantitatifs de ces deux essais.

Tableau 6

| Essai | Taux de transformation en produits hydroxylés | Proportions relatives des isomères ortho | méta | para |
|---|---|---|---|---|
| 6a | 64% | 0 | 44 | 56 |
| 6b | 81% | 2 | 46 | 52 |

Exemple 7

Hydroxylation du carbonate de méthyle et de phényle en milieu $HF-BF_3$

a) On introduit dans le réacteur 18 millimoles de carbonate de méthyle et de phényle. On refroidit à —60° C, fait le vide et injecte 1,5 mole d'acide fluorhydrique anhydre, puis 1,5 mole de trifluorure de bore. A l'aide d'une pompe on introduit ensuite 2 équivalents de peroxyde d'hydrogène, sous forme d'une solution aqueuse à 70% en poids.

Après 30 minutes de réaction à —50° C sous agitation, on traite les produits de réaction comme indiqué au mode opératoire général.

b) On opère comme ci-dessus, mais la température de réaction est de —45° C.

Dans les deux cas les analyses montrent qu'il s'est formé uniquement les trois isomères ortho, méta et para du carbonate de méthyle et d'hydroxyphényle.

Le tableau 7 rassemble les résultats quantitatif obtenus.

Tableau 7

| Essai | Taux de transformation en produits hydroxylés | Proportions relatives des isomères ortho | méta | para |
|---|---|---|---|---|
| 7a | 9% | 3 | 46 | 51 |
| 7b | 12% | 3 | 47 | 50 |

Exemple 8

Hydroxylation du carbonate de bis-phényle en milieu $HF-SbF_5$

a) A 12 ml d'un mélange $HF-SbF_5$ 1,75 M (rapport molaire $SbF_5/HF = 0,04$) refroidi à —40° C, on ajoute successivement 2 millimoles de carbonate de bis-phényle et 1,5 équivalent de peroxyde d'hydrogène sous forme d'une solution aqueuse à 95% en poids. Après 30 minutes de réaction, le mélange réactionnel est traité comme indiqué au mode opératoire général. Les produits de la réaction sont élués par un mélange 85/15 en volumes d'éther de pétrole et d'acétate d'éthyle, qui sort successivement les produits suivants, identifiés par leurs spectres infra-rouges et leurs spectres de résonance magnétique nucléaire:

- carbonate de bis-phényle,
- phénol,
- carbonate de phényle et d'hydroxy-3-phényle,
- carbonate de phényle et d'hydroxy-4-phényle,
- résorcinol,
- hydroquinone.

b) On opère comme ci-dessus, mais à la température de 0° C pendent 5 minutes seulement.

Le tableau 8 donne les rendements pondéraux des produits obtenus.

Tableau 8

| Essai | Carbonate de bis-phényle non transformé | Phénol | Carbonate de phényle et d'hydroxy-3-phényle | Carbonate de phényle et d'hydroxy-4-phényle | Résor-cinol | Hydro-qinone |
|---|---|---|---|---|---|---|
| 8a | 24 | 5 | 25 | 28 | 11 | 2 |
| 8b | 23 | 4 | 20 | 25 | — | — |

Exemple 9

Hydroxylation du benzoate de phényle en milieu HF—$SbF_5$.

A 20 ml d'un mélange HF—$SbF_5$ 1,75 M (rapport molaire $SbF_5$/HF = 0,04) refroidi à —20° C, on ajoute 3 millimole de benzoate de phényle, puis 2 équivalentes de peroxyde l'hydrogène sous forme d'une solution aqueuse à 70% en poids. Après 30 minutes de réaction à —20° C, le mélange réactionnel est traité de la manière habituelle. Les produits bruts sont chromatographiés sur gel de silice. Le mélange éther de pétrole — acétate d'éthyle à 87,5/12,5 en volumes élue successivement les produits suivant, dont la structure découle des caractéristiques spectroscopiques (Résonance magnétique nucléaire et spectrographie de masse):

— benzoate de phényle non transformé,
— monobenzoate de pyrocatéchol, ou benzoate d'hydroxy-2-phényle,
— monobenzoate de résorcinol, ou benzoate d'hydroxy-3-phényle,
— monobenzoate d'hydroquinone, ou benzoate d'hydroxy-4-phényle.

Dans le tableau 9 figurent les résultats quantitatifs obtenus.

Tableau 9

| Taux de transformation en produits hydroxylés | Proportions relatives des isomères ortho | méta | para |
|---|---|---|---|
| 80% | 23 | 30 | 47 |

Exemple 10

Hydroxylation du p-nitrobenzoate de phényle en milieu HF—$SbF_5$

A 16 ml d'un mélange HF—$SbF_5$ 1,75 M (rapport molaire $SbF_5$/HF = 0,04) refroidi à —20° C, on ajoute 2,5 millimoles de p-nitrobenzoate de phényle et 2 équivalente de peroxyde d'hydrogène sous forme d'une solution aqueuse à 70% en poids. Au bout de 30 minutes de réaction, le mélange est hydrolysé et extrait. Les produits de la réaction sont chromatographiés sur gel de silice. Le mélange éther de pétroleacétate d'éthyle 85/15 en volumes élue successivement:

— le p-nitrobenzoate de phényle non transformé,
— le p-nitrobenzoate d'hydroxy-2-phényle,
— le p-nitrobenzoate d'hydroxy-3-phényle,
— le p-nitrobenzoate d'hydroxy-4-phényle.

Le tableau 10 donne les résultats quantitatifs de cet essai.

Tableau 10

| Taux de transformation en produits hydroxylés | Proportions relatives des isomères ortho | méta | para |
|---|---|---|---|
| 66% | 15 | 38 | 47 |

Exemple 11

Hydroxylation du formanilide en milieu HF—$SbF_5$

On opère comme dans l'exemple 10, mais en remplaçant le p-nitrobenzoate de phényle par 4 millimoles de formanilide. La durée de réaction est de 20 minutes. Après traitement, les produits de la réaction sont chromatographiés sur gel de silice. Le mélange éther de pétrole — acétate d'éthyle 50/50 en volumes élue successivement:

— le formanilide non transformé,
— l'hydroxy-2-formanilide,
— l'hydroxy-3-formanilide,
— l'hydroxy-4-formanilide.

Le tableau 11 donne les résultats quantitatifs de cet essai.

Tableau 11

| Taux de transformation en produits hydroxylés | Proportions relatives des isomères ortho | méta | para |
|---|---|---|---|
| 76% | 26 | 25 | 49 |

Exemple 12

Hydroxylation de l'acétanilide en milieu HF—$SbF_5$

A 14 ml d'un mélange HF—$SbF_5$ 1,75 M (rapport molaire $SbF_5$/HF = 0,04) refroid à —40° C, on ajoute 3 millimoles d'acétanilide, puis 2 équivalents de peroxyde d'hydrogène sous forme d'une solution aqueuse à 70% en poids. Le mélange réactionnel est maintenu pendant 30 minutes à —40° C, puis traité de la manière habituelle.

Les produits de la réaction sont chromatographiés sur gel de silice, et le mélange acétate d'éthyle — éther de pétrole 75/25 élue successivement:

— l'hydroxy-2-acétanilide, ou acétamino-2-phénol,
— l'acétanilide non transformé,
— l'hydroxy-3-acétanilide,
— l'hydroxy-4-acétanilide.

Les résultats quantitatifs de cet essai sont consignés dans le tableau 12.

Tableau 12

| Taux de transformation en produits hydroxylés | Proportions relatives des isomères ortho | méta | para |
|---|---|---|---|
| 74% | 37 | 29 | 34 |

## Exemple 13

### Hydroxylation du benzanilide en milieu HF—$SbF_5$

a) A 15 ml d'un mélange HF—$SbF_5$ 1,75 M (rapport molaire $SbF_5$/HF = 0,04) refroidi à —20°C, on ajoute 2 millimoles de benzanilide et 1,5 équivalent de peroxyde d'hydrogène sous forme d'une solution aqueuse à 70% en poids. Après 30 minutes de réaction à —20°C, le mélange réactionnel est soumis à l'hydrolyse et à l'extraction.

Les produits de réaction sont chromatographiés sur gel de silice, et le mélange éther de pétrole — acétate d'éthyle 75/25 en volume élue successivement:

- le benzanilide non transformé,
- l'hydroxy-2-benzanilide,
- l'hydroxy-3-benzanilide,
- l'hydroxy-4-benzanilide.

On note la formation d'une petite quantité de dérivés dihydroxylés, qui n'ont pas été autrement caractérisés.

b) On opère comme ci-dessus, mais la concentration de $SbF_5$ dans l' HF est 2 M (rapport molaire $SbF_5$/HF = 0,05).

Le tableau 13 rassemble les résultats quantitatifs de ces deux essais.

Tableau 13

| Essai | Taux de transformation en dérivés monohydroxylés | en dérivés dihydroxylés | Proportions relatives des isomères monohydroxylés ortho | méta | para |
|---|---|---|---|---|---|
| 13a | 71% | ~12% | 41 | 27 | 32 |
| 13b | 69% | ~ 7% | 42 | 29 | 29 |

## Exemple 14

### Hydroxylation du phénylcarbamate de méthyle en milieu HF—$SbF_5$

A 16 ml d'un mélange HF—$SbF_5$ 1,75 M (rapport molaire $SbF_5$/HF = 0,04) refroidi à —40°C, on ajoute 3 millimoles de phénylcarbamate de méthyle, puis 2 équivalents de peroxyde d'hydrogène sous forme d'une solution aqueuse à 70% en poids. Après 30 minutes de réaction à —40°C, le mélange est traité de la manière habituelle et les produits de réaction sont chromatographiés sur gel de silice. Le mélange éther de pétrole — acétate d'éthyle 75/25 en volume élue successivement:

- le phénylcarbamate de méthyle non transformé,
- l'hydroxy-2-phénylcarbamate de méthyle,
- l'hydroxy-3-phénylcarbamate de méthyle,
- l'hydroxy-4-phénylcarbamate de méthyle.

Le tableau 14 donne les résultats quantitatifs de cet essai.

Tableau 14

| Taux de transformation en produits hydroxylés | Proportions relatives des isomères ortho | méta | para |
|---|---|---|---|
| 83% | 30 | 24 | 46 |

Exemple 15

Hydroxylation du phénylcarbamate de phényle en milieu HF—$SbF_5$

On opère comme dans l'exemple précédent, mais en remplaçant le phénylcarbamate de méthyle par 2 millimoles de phénylcarbamate de phényle. On utilise pour la chromatographie sur gel de silice un mélange éther de pétrole — acétate d'éthyle 80/20 en volume, qui élue successivement:

— le phénylcarbamate de phényle non transformé,
— le N-(hydroxy-2-phényl)-carbamate de phényle,
— le N-(hydroxy-3-phényl)-carbamate de phényle,
— le N-(hydroxy-4-phényl)-carbamate de phényle,

identifiés par leurs spectres de résonance magnétique nucléaire et de masse.

On observe la formation de 23% environ de produits plus polaires non identifiés.

Dans le tableau 15 sont donnés les résultats quantitatifs de cet essai.

Tableau 15

| Taux de transformation en produits hydroxylés | Proportions relatives des isomères ortho | méta | para |
|---|---|---|---|
| 65% | 36 | 22 | 42 |

**Revendications**

1. Procédé d'hydroxylation de dérivés du phénol et de l'aniline par le peroxyde d'hydrogène, donnant une proportion importante de dérivé monohydroxylé en méta, caractérisé en ce que l'on fait réagir le peroxyde d'hydrogène dans un milieu liquide superacide, constitué de produits qui sur l'échelle logarithmique de HAMMET ont des activités atteignant environ —25, à des températures allant d'environ —80° C environ 0° C, sur des composés répondant à la formule générale:

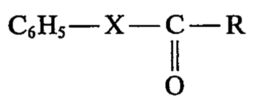

dans laquelle X représente un atome d'oxygène ou un radical —NH— et R représente un atome d'hydrogène, ou un groupement alkyle contenant de 1 à 8 atomes de carbone, ou un groupement cycloalkyle contenant de 5 à 10 atomes de carbone, ou un groupement aryle, éventuellement substitué, ou un groupement alcoxy contenant de 1 à 8 atomes de carbone, ou un groupement cycloalcoxy contenant de 5 à 10 atomes de carbone ou un groupement aryloxy.

2. Procédé selon la revendication 1, caractérisé en ce que le milieu superacide liquide est constitué par l'acide fluoroantimonique HF—$SbF_5$.

3. Procédé selon la revendication 1, caractérisé en ce que le milieu superacide liquide est constitué par le complexe acide fluorhydrique anhydre-trifluorure de bore HF—$BF_3$.

4. Procédé selon la revendication 1, caractérisé en ce que le milieu superacide liquide est constitué par le complexe acide fluorosulfonique-pentafluorure d'antimoine $FSO_3H$—$SbF_5$.

5. Procédé selon la revendication 1, caractérisé en ce que le milieu superacide liquide est constitué par le complexe acide trifluorométhanesulfonique-pentafluorure d'antimoine $CF_3SO_3H$—$SbF_5$.

6. Procédé selon l'une quelconque des revendications 1 à 5, caractérisé en ce que le peroxyde d'hydrogène est mis en œuvre sous forme d'une solution aqueuse contenant de 20 à 98% en poids de peroxyde d'hydrogène.

7. Procédé selon la revendication 6, caractérisé en ce que le peroxyde d'hydrogène est utilisé en excès allant de 10 à 100% par rapport à la stoechiometrie.

8. Procédé selon la revendication 1, caractérisé en ce que le composé à hydroxyler est un ester du phénol avec un monoacide ou un polyacide carboxylique.

9. Procédé selon la revendication 1, caractérisé en ce que le composé à hydroxyler est le carbonate de bis-phényle.

10. Procédé selon la revendication 1, caractérisé en ce que le composé à hydroxyler est un carbonate mixte de phényle et d'alkyle, ou de cycloalkyle ou d'aryle substitué.

11. Procédé selon la revendication 1, caractérisé en ce que le composé à hydroxyler est un anilide.

12. Procédé selon la revendication 1, caractérisé en ce que le composé à hydroxyler est un phénylcarbamate d'alkyle, de cycloalkyle ou d'aryle.

**Patentansprüche**

1. Verfahren zur Hydroxylierung von Derivaten des Phenols und des Anilins mit Wasserstoffperoxid, das einen wesentlichen Anteil an dem in Meta-Stellung substituierten Monohydroxyl-Derivat liefert, dadurch gekennzeichnet, daß man in einem flüssigen supersauren Milieu, das aus Produkten besteht, die auf der logarithmischen Hammet-Skala Aktivitäten von etwa —25 erreichen, bei Temperaturen zwischen etwa —80°C und etwa 0°C Wasserstoffperoxid mit Verbindungen der allgemeinen Formel umsetzt:

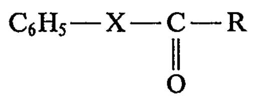

in der X ein Sauerstoffatom oder ein —NH-Radikal, und R ein Wasserstoffatom oder eine Alkylgruppe mit 1 bis 8 Kohlenstoffatomen, eine Cycloalkylgruppe mit 5 bis 10 Kohlenstoffatomen, eine gegebenenfalls substituierte Arylgruppe, eine Alkoxygruppe mit 1 bis 8 Kohlenstoffatomen, eine Cycloalkoxygruppe mit 5 bis 10 Kohlenstoffatomen oder eine Aryloxygruppe bedeuten.

2. Verfahren nach Anspruch 1, dadurch gekennzeichnet, daß das flüssige supersaure Milieu aus der Fluoroantimonsäure $HF-SbF_5$ besteht.

3. Verfahren nach Anspruch 1, dadurch gekennzeichnet, daß das flüssige supersaure Milieu aus dem Komplex zwischen wasserfreier Fluorwasserstoffsäure und Bortrifluorid $HF-BF_3$ besteht.

4. Verfahren nach Anspruch 1, dadurch gekennzeichnet, daß das flüssige supersaure Milieu aus dem Komplex zwischen Fluorsulfonsäure und Antimonpentafluorid $FSO_3H-SbF_5$ besteht.

5. Verfahren nach Anspruch 1, dadurch gekennzeichnet, daß das flüssige supersaure Milieu aus dem Komplex zwischen Trifluormethansulfonsäure und Antimonpentafluorid $CF_3SO_3H-SbH_5$ besteht.

6. Verfahren nach einem der Ansprüche 1 bis 5, dadurch gekennzeichnet, daß das Wasserstoffperoxid als wäßrige Lösung mit 20 bis 98 Gew.-% Wasserstoffperoxid eingesetzt wird.

7. Verfahren nach Anspruch 6, dadurch gekennzeichnet, daß das Wasserstoffperoxid in einem Überschuß zwischen 10 und 100%, bezogen auf die stöchiometrischen Menge, eingesetzt wird.

8. Verfahren nach Anspruch 1, dadurch gekennzeichnet, daß die zu hydroxylierende Verbindung ein Ester des Phenols mit einer ein- oder mehrwertigen Carbonsäure ist.

9. Verfahren nach Anspruch 1, dadurch gekennzeichnet, daß die zu hydroxylierende Verbindung Bisphenylcarbonat ist.

10. Verfahren nach Anspruch 1, dadurch gekennzeichnet, daß die zu hydroxylierende Verbindung ein gemischtes Phenyl-/Alkyl-Carbonat, Phenyl-/Cycloalkyl- oder Phenyl-/substituiertes Aryl-Carbonat ist.

11. Verfahren nach Anspruch 1, dadurch gekennzeichnet, daß die zu hydroxylierende Verbindung ein Anilid ist.

12. Verfahren nach Anspruch 1, dadurch gekennzeichnet, daß die zu hydroxylierende Verbindung ein Alkyl-, Cycloalkyl- oder Aryl-Phenyl-Carbamat ist.

**Claims**

1. Process for the hydroxylation of phenol and aniline derivatives with hydrogen peroxide, yielding a high proportion of meta-monohydroxylated derivatives, characterised in that hydrogen peroxide is reacted in a superacid liquid medium consisting of products which have activities of up to about —25 on the Hammett logarithmic scale, at temperatures ranging from approximately —80°C to approximately 0°C, with compounds corresponding to the general formula:

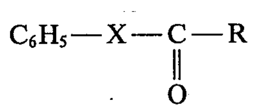

in which X denotes an oxygen atom or an —NH— radial and R denotes a hydrogen atom, or an alkyl group containing from 1 to 8 carbon atoms, or a cycloalkyl group containing from 5 to 10 carbon atoms, or an optionally substituted aryl group, or an alkoxy group containing from 1 to 8 carbon atoms, or a cycloalkoxy group containing from 5 to 10 carbon atoms or an aryloxy group.

2. Process according to Claim 1, characterised in that the liquid superacid medium consists of fluoroantimonic acid $HF-SbF_5$.

3. Process according to Claim 1, characterised in that the liquid superacid medium consists of the anhydrous hydrofluoric acid-boron trifluoride complex $HF-BF_3$.

4. Process according to Claim 1, characterised in that the liquid superacid medium consists of the fluorosulphonic acid-antimony pentafluoride complex $FSO_3H-SbF_5$.

5. Process according to Claim 1, characterised in that the liquid superacid medium consists of the trifluoromethanesulphonic acid-antimony pentafluoride complex $CF_3SO_3H-SbF_5$.

6. Process according to any one of Claims 1 to 5, characterised in that hydrogen peroxide is used in the form of an aqueous solution containing from 20 to 98% weight of hydrogen peroxide.

7. Process according to Claim 6, characterised in that hydrogen peroxide is employed in an excess ranging from 10 to 100% relative to the stoichiometry.

8. Process according to Claim 1, characterised in that the compound to be hydroxylated is an ester of phenol with a monocarboxylic or polycarboxylic acid.

9. Process according to Claim 1, characterised in that the compound to be hydroxylated is bisphenyl carbonate.

10. Process according to Claim 1, characterised in that the compound to be hydroxylated is a mixed carbonate which is a phenyl carbonate together weith an alkyl, cycloalkyl or substituted aryl-carbonate.

11. Process according to Claim 1, characterised in that the compound to be hydroxylated an anilide.

12. Process according to Claim 1, characterised in that the compound to be hydroxylated is an alkyl, cycloalkyl or aryl phenylcarbamate.